(19) Europäisches Patentamt
European Patent Office
Office européen des brevets

(11) EP 3 075 379 A1

(12) **EUROPEAN PATENT APPLICATION**
published in accordance with Art. 153(4) EPC

(43) Date of publication:
**05.10.2016 Bulletin 2016/40**

(21) Application number: **14835076.2**

(22) Date of filing: **07.08.2014**

(51) Int Cl.:
***A61K 31/05*** (2006.01) ***A61K 31/353*** (2006.01)
***A61K 36/00*** (2006.01) ***A61K 36/18*** (2006.01)
***A61K 36/48*** (2006.01) ***A61K 36/73*** (2006.01)
***A61P 3/06*** (2006.01) ***A61P 3/10*** (2006.01)
***A61P 9/12*** (2006.01) ***A61P 29/00*** (2006.01)
***A61P 31/04*** (2006.01) ***A61P 39/06*** (2006.01)
***A61P 43/00*** (2006.01)

(86) International application number:
**PCT/JP2014/070839**

(87) International publication number:
**WO 2015/020138 (12.02.2015 Gazette 2015/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.08.2013 JP 2013166606**
**21.04.2014 JP 2014087023**

(71) Applicant: **Suntory Holdings Limited**
**Osaka-shi, Osaka 530-8203 (JP)**

(72) Inventors:
- **UENO, Toshiya**
  **Soraku-gun**
  **Kyoto 619-0284 (JP)**
- **KOMINAMI, Masaru**
  **Soraku-gun**
  **Kyoto 619-0284 (JP)**
- **KASAJIMA, Naoki**
  **Soraku-gun**
  **Kyoto 619-0284 (JP)**

(74) Representative: **Vossius & Partner**
**Patentanwälte Rechtsanwälte mbB**
**Siebertstrasse 3**
**81675 München (DE)**

# (54) AGENT FOR PROMOTING IN VIVO ABSORPTION OF HYDROXYTYROSOL AND DERIVATIVES THEREOF AND USE OF SAME

(57) The purpose of the present invention is to provide a means for promoting the in vivo absorption of hydroxytyrosol or a derivative thereof, prolonging the in vivo residence time, and sustaining the effect. As a result of in-depth studies, the present inventors discovered that the in vivo absorption of hydroxytyrosol or a derivative thereof can be promoted and the in vivo residence time can be prolonged by using hydroxytyrosol or a derivative thereof in combination with a compound having a galloyl group.

EP 3 075 379 A1

## Description

TECHNICAL FIELD

The present invention relates to compositions for promoting the absorption in the body of hydroxytyrosol and derivatives thereof, agents for promoting the absorption in the body of hydroxytyrosol and derivatives thereof or for prolonging their residence time in the body, as well as foods and beverages or pharmaceutical products using said compositions or agents.

BACKGROUND ART

Hydroxytyrosol is one type of polyphenols contained in olive. Hydroxytyrosol is known to have various physiological activities beneficial for the living body, including strong anti-oxidant and anti-inflammatory activities. It has also been recently reported that Hydroxytyrosol is useful as an anti-aging agent (Patent Literature 1).

However, there are problems with hydroxytyrosol, in that it is low in absorption in the body and bioavailability. Hydroxytyrosol is also problematic in that since it clears rapidly from the body, its residence time in the body is short and its effects do not last long.

Hitherto, various attempts have been made to increase the absorption of polyphenols in the body or to prolong their residence time in the body. For example, it was reported that the absorption and residence time of catechins in the body can be improved by consuming catechins concurrently with an extract of *"Benifuuki",* a tea cultivar (Patent Literature 2). Another report stated that serine, asparatic acid, malic acid, capric acid, lauric acid, grapefruit juice, succinic acid, cysteine, asparagine, isoleucine, and pinitol are capable of promoting the absorption of epigallocatechin gallate and increasing its residence time in the body (Patent Literature 3).

However, as for the particular polyphenols, *i.e.,* hydroxytyrosol and derivatives thereof, no example has been reported of a compound or composition that is capable of improving their absorption in the body and prolonging their residence time in the body.

CITATION LIST

PATENT LITERATURES

Patent Literature 1: Japanese Patent Domestic Publication No. JP 2010-52487
Patent Literature 2: Japanese Patent Application Publication No. JP 2010-11751
Patent Literature 3: Japanese Patent Application Publication No. JP 2011-79770

SUMMARY OF INVENTION

TECHNICAL PROBLEM

In view of the aforementioned problems with the prior art, an object of the present invention is to provide a means for promoting the absorption in the body of hydroxytyrosol or a derivative thereof, prolonging its residence time in the body, and sustaining its effects.

SOLUTION TO PROBLEM

The present inventors have made extensive studies to solve the aforementioned problems and, as a result, have found that the use of hydroxytyrosol in combination with a compound having a galloyl group allows promotion of the absorption of hydroxytyrosol in the body and prolongation of its residence time in the body.

More specifically, the present invention is directed to the following.

[1] A composition comprising hydroxytyrosol or a derivative thereof, and a compound having a galloyl group.
[2] The composition as set forth in [1], wherein the compound having a galloyl group is a monomer of a flavane compound having one or more galloyl groups in a flavane backbone.
[3] The composition as set forth in [2], wherein the monomer is a flavane compound having a galloyl group attached to position 3 of the flavane backbone.
[4] The composition as set forth in [2], wherein the compound having a galloyl group is a catechin compound having

a galloyl group.
[5] The composition as set forth in [4], wherein the catechin compound having a galloyl group is a catechin compound comprising at least one member selected from the group consisting of catechin gallate, epicatechin gallate, gallocatechin gallate, epigallocatechin gallate and methylated derivatives thereof.
[6] The composition as set forth in [1], wherein the compound having a galloyl group is a polymer of a flavane compound having a flavane backbone, wherein at least one monomeric unit is a flavane compound having a galloyl group attached to position 3 of the flavane backbone.
[7] The composition as set forth in [6], wherein the polymer is an oligomeric proanthocyanidin.
[8] The composition as set forth in any one of [1] to [7], wherein the compound having a galloyl group is derived from grape, pine, aronia, peanut, cocoa, apple, red bean, tamarind, persimmon, green tea, or black tea.
[9] The composition as set forth in [8], wherein the compound having a galloyl group is derived from a grape seed extract.
[10] A composition comprising hydroxytyrosol or a derivative thereof, and a grape seed extract.
[11] The composition as set forth in any one of [1] to [9], wherein the hydroxytyrosol or the derivative thereof, and the compound having a galloyl group are present at a weight ratio of from 1:0.1 to 1:100.
[12] The composition as set forth in [11], wherein the hydroxytyrosol or the derivative thereof, and the compound having a galloyl group are present at a weight ratio of from 1:0.1 to 1:30.
[13] The composition as set forth in [11] or [12], wherein the compound having a galloyl group is a monomer of a flavane compound having one or more galloyl groups in a flavane backbone.
[14] The composition as set forth in [11], wherein the hydroxytyrosol or the derivative thereof, and the oligomeric proanthocyanidins are present at a weight ratio of from 1:1 to 1:100.
[15] The composition as set forth in any one of [1] to [9], wherein the compound having a galloyl group is a galloyl ester which produces a gallic acid by acid hydrolysis, and wherein the hydroxytyrosol or the derivative thereof, and the gallic acid produced by acid hydrolysis of the galloyl ester are present at a molar ratio of from 1:0.1 to 1:10.
[16] The composition as set forth in any one of [1] to [10], wherein the hydroxytyrosol or the derivative thereof is present in a relative amount of from 0.01 to 10% by weight, and the compound having a galloyl group is present in a relative amount of from 0.1 to 30% by weight.
[17] The composition as set forth in any one of [1] to [16], wherein the composition is a food or beverage.
[18] The composition as set forth in any one of [1] to [16], wherein the composition is a pharmaceutical composition.
[19] An agent for promoting absorption in the body of hydroxytyrosol or a derivative thereof, the agent comprising, as an active component, a compound having a galloyl group.
[20] The agent as set forth in [19], wherein the compound having a galloyl group is a monomer of a flavane compound having one or more galloyl groups in a flavane backbone.
[21] The agent as set forth in [20], wherein the monomer is a flavane compound having a hydroxyl group attached to position 3 of the flavane backbone, or is a flavane compound having a galloyl group attached to position 3 of the flavane backbone.
[22] The agent as set forth in [20], wherein the compound having a galloyl group is a catechin compound having a galloyl group.
[23] The agent as set forth in [22], wherein the catechin compound having a galloyl group is a catechin compound comprising at least one member selected from the group consisting of catechin gallate, epicatechin gallate, gallocatechin gallate, epigallocatechin gallate and methylated derivatives thereof.
[24] The agent as set forth in [19], wherein the compound having a galloyl group is a polymer of a flavane compound having a flavane backbone, wherein at least one monomeric unit is a flavane compound having a galloyl group attached to position 3 of the flavane backbone.
[25] The agent as set forth in [24], wherein the polymer is an oligomeric proanthocyanidin.
[26] The agent as set forth in any one of [19] to [25], wherein the compound having a galloyl group is derived from grape, pine, aronia, peanut, cocoa, apple, red bean, tamarind, persimmon, green tea, or black tea.
[27] The agent as set forth in [26], wherein the compound having a galloyl group is derived from a grape seed extract.
[28] An agent for promoting absorption in the body of hydroxytyrosol or a derivative thereof, the agent comprising a grape seed extract.
[29] The agent as set forth in any one of [19] to [28], wherein the compound having a galloyl group is present in a relative amount of 0.1 to 30% by weight.
[30] The agent as set forth in any one of [19] to [29], wherein the compound having a galloyl group is a galloyl ester which produces a gallic acid by acid hydrolysis, wherein the galloyl ester is present in a relative amount of 0.01 to 25% by weight in terms of the weight of the gallic acid produced by acid hydrolysis of the galloyl ester.
[31] The agent as set forth in any one of [19] to [28], wherein the compound having a galloyl group is a galloyl ester which produces a gallic acid by acid hydrolysis, wherein the relative amount of the galloyl ester present per gram of the agent is in the range of from 0.001 to 1 mmol in terms of the gallic acid produced by acid hydrolysis of the

galloyl ester.

[32] The agent as set forth in any one of [25] to [29], wherein the oligomeric proanthocyanidin is present in a relative amount of 0.1 to 30% by weight.

[33] An agent for prolonging residence time in the body ofhydroxytyrosol or a derivative thereof, the agent comprising, as an active component, a compound having a galloyl group.

[34] The agent as set forth in [33], wherein the compound having a galloyl group is a monomer of a flavane compound having one or more galloyl groups in a flavane backbone.

[35] The agent as set forth in [34], wherein the monomer is a flavane compound having a hydroxyl group attached to position 3 of the flavane backbone, or is a flavane compound having a galloyl group attached to position 3 of the flavane backbone.

[36] The agent as set forth in [34], wherein the compound having a galloyl group is a catechin compound having a galloyl group.

[37] The agent as set forth in [36], wherein the catechin compound having a galloyl group is a catechin compound comprising at least one member selected from the group consisting of catechin gallate, epicatechin gallate, gallocatechin gallate, epigallocatechin gallate and methylated derivatives thereof.

[38] The agent as set forth in [33], wherein the compound having a galloyl group is a polymer of a flavane compound having a flavane backbone, wherein at least one monomeric unit is a flavane compound having a galloyl group attached to position 3 of the flavane backbone.

[39] The agent as set forth in [38], wherein the polymer is an oligomeric proanthocyanidin.

[40] The agent as set forth in any one of [33] to [39], wherein the compound having a galloyl group is derived from grape, pine, aronia, peanut, cocoa, apple, green tea, or black tea.

[41] The agent as set forth in [40], wherein the compound having a galloyl group is derived from a grape seed extract.

[42] An agent for prolonging residence time in the body of hydroxytyrosol or a derivative thereof, the agent comprising a grape seed extract.

[43] The agent as set forth in any one of [33] to [42], wherein the compound having a galloyl group is present in a relative amount of 0.1 to 30% by weight.

[44] The agent as set forth in any one of [33] to [43], wherein the compound having a galloyl group is a galloyl ester which produces a gallic acid by acid hydrolysis, wherein the galloyl ester is present in a relative amount of 0.01 to 25% by weight in terms of the weight of the gallic acid produced by acid hydrolysis of the galloyl ester.

[45] The agent as set forth in any one of [33] to [43], wherein the compound having a galloyl group is a galloyl ester which produces a gallic acid by acid hydrolysis, wherein the relative amount of the galloyl ester present per gram of the agent is in the range of from 0.001 to 1 mmol in terms of the gallic acid produced by acid hydrolysis of the galloyl ester.

[46] The agent as set forth in any one of [38] to [43], wherein the oligomeric proanthocyanidin is present in a relative amount of from 0.1 to 30% by weight.

## ADVANTAGEOUS EFFECTS OF INVENTION

According to the present invention, the absorption in the body of hydroxytyrosol or a derivative thereof can be improved and its residence time in the body can also be prolonged by using the hydroxytyrosol or the derivative thereof in combination with a compound having a galloyl group.

The compound having a galloyl group is commonly a plant-derived compound such as flavanes and thus is very high in safety. In particular, catechins typically contained in tea have various physiological activities including anti-oxidant activity, blood pressure elevation inhibitory activity, blood sugar elevation inhibitory activity, body fat accumulation inhibitory activity, and anti-microbial activity. Also, oligomeric proanthocyanidins (hereinafter also referred to as simply "proanthocyanidins" or as "OPCs") which are contained in grape and pine have, as a monomeric unit, a flavane compound having a galloyl group. OPC's are known to have anti-oxidant, blood flow-improving and other activities. Therefore, the present invention can be expected not only to allow improvement of the absorption in the body of hydroxytyrosol or a derivative thereof and prolongation of its residence time in the body, but also to help a flavane compound having a galloyl group to exhibit its beneficial physiological activities, as well as makes it possible to provide safe and continuously ingestible pharmaceutical compositions, foods and beverages.

## BRIEF DESCRIPTION OF DRAWINGS

FIG. 1 shows the amount of hydroxytyrosol (HT) absorbed in the body (AUC).

FIG. 2 shows the influence of EGCG on change in blood hydroxytyrosol (HT) concentration over time.

FIG. 3 shows the amount of hydroxytyrosol (HT) absorbed in the body (AUC).

FIG. 4 shows the transition of blood hydroxytyrosol (HT) concentration.

FIG. 5 shows the amount of hydroxytyrosol (HT) absorbed in the body (AUC).

FIG. 6 shows the amount of hydroxytyrosol (HT) absorbed in the body (AUC), the molar ratio of HT to a galloyl ester, and the weight ratio of HT to OPCs. The HT/galloyl ester molar ratio was calculated as a ratio of the number of moles of HT to that of a gallic acid produced by acid hydrolysis of the galloyl ester.

FIG. 7 shows the transition of blood hydroxytyrosol (HT) concentration.

DESCRIPTION OF EMBODIMENTS

The present invention relates to a composition comprising hydroxytyrosol or a derivative thereof, and a compound having a galloyl group, and to agents for promoting the absorption in the body of hydroxytyrosol or a derivative thereof or for prolonging its residence time in the body.

<Hydroxytyrosol>

The composition of the present invention comprises hydroxytyrosol or a derivative thereof, and a compound having a galloyl group. Also, this invention relates to agents for promoting the absorption in the body of hydroxytyrosol or a derivative thereof or for prolonging its residence time in the body.

Hydroxytyrosol (3,4-dihydroxyphenylethanol) is one type of polyphenol compounds, and is represented by the structure shown below.

[Chemical Formula 1]

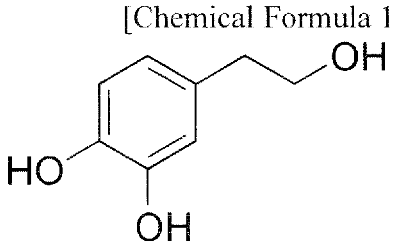

In the present invention, it is acceptable to use a chemically synthesized hydroxytyrosol, a hydroxytyrosol-rich plant ingredient as it is, a hydroxytyrosol-containing extract from a plant ingredient like olive, or a product having its hydroxytyrosol content being increased by purifying such a hydroxytyrosol-containing extract.

In the present invention, a derivative of hydroxytyrosol can also be used. Nonlimiting examples of the hydroxytyrosol derivative include esters such as carboxylic acid ester, sulfonic acid ester, phosphoric acid ester, phosphonic acid ester, and amino acid ester, and salts such as hydrochloride, phosphate, sulfate, acetate, citrate, succinate, sodium salt, potassium salt, calcium salt, and ammonium salt.

A chemically synthesized hydroxytyrosol is available from, for example, Tokyo Chemical Industry Co., Ltd.

A plant ingredient rich in hydroxytyrosol may be directly incorporated in the composition of the present invention. As the plant ingredient rich in hydroxytyrosol, leaves, fruits, seeds, stems and other portions of olive, for example, can be used raw or in a dried state achieved by freeze-drying or other means. Any variety of olive can be used but, for example, olives of the following varieties can be used preferably: Manzanillo, Lucca, Nevadillo blanco, Mission, Picual, Arbequina, Hojiblanca, Cornicabra, Gordal, Moraiolo, Frantoio, Coratina, and Leccino. Also, an olive oil obtained by pressing olive fruits can be used as the plant ingredient rich in hydroxytyrosol. A commercially available olive oil may be used, or an olive oil may be prepared from olive fruits by any known method.

Alternatively, a hydroxytyrosol-containing extract from a plant ingredient like olive may be incorporated in the composition of the present invention. For example, the hydroxytyrosol-containing extract can be obtained by providing the aforementioned plant ingredient in a raw or dried state and subjecting said plant ingredient, as it is or after crushed by a coarse grinder, to extraction with an aqueous solvent. A commercially available olive leaf extract may also be used as the hydroxytyrosol-containing extract.

<Compound having a galloyl group>

The composition of the present invention comprises hydroxytyrosol or a derivative thereof, and a compound having a galloyl group. Also, the inventive agents for promoting the absorption in the body of hydroxytyrosol or a derivative thereof or for prolonging its residence time in the body comprise a compound having a galloyl group.

The compound having a galloyl group as used in the present invention is preferably a monomer of a flavane

compound having one or more galloyl groups in a flavane backbone, or a polymer of a flavane compound having a flavane backbone, wherein at least one monomeric unit is a flavane compound having a galloyl group attached to position 3 of the flavane backbone. In one mode of this invention, the compound having a galloyl group is a galloyl ester obtained by condensation reaction of a compound having one or more hydroxyl groups in the flavane backbone with a gallic acid. In other words, in one mode of this invention, the compound having a galloyl group is a galloyl ester which produces a gallic acid by acid hydrolysis of said compound.

Examples of the monomer of the flavane compound having one or more galloyl groups in a flavane backbone include, but are not limited to, a flavane compound having a galloyl group attached to position 3 of a flavane backbone.

Examples of the flavane compound having a galloyl group attached to position 3 of a flavane backbone include flavanols, flavonols, anthocyanins and chalcones, which have a galloyl group at position 3. The flavanols having a galloyl group at position 3 can be exemplified by catechin gallate, epicatechin gallate, gallocatechin gallate, epigallocatechin gallate and methylated derivatives thereof. Among them, epigallocatechin gallate and methylated derivatives thereof can be mentioned as particularly preferred examples.

Examples of the polymer of the flavane compound having a flavane backbone, wherein at least one monomeric unit is a flavane compound having a galloyl group attached to position 3 of the flavane backbone, include oligomeric proanthocyanidins (OPCs). OPCs are one type of polyphenols abundant in grape, apple, cacao and other plants, and are also called "condensed tannins" or "flavan-3-ol polymers". Speaking of the structure of OPCs, the monomeric units, flavan-3-ols, are generally bonded together by condensation or polymerization at the C4-C6 or C4-C8 position. Thus, OPCs are a generic name for a dimer and higher polymers produced by condensation or polymerization according to the bonding method mentioned above.

The compound having a galloyl group as used in the present invention is by no means limited by its form, preparation method, or the like, but for example, those compounds which are derived from plants such as grape, pine, aronia, peanut, cocoa, apple, red bean, tamarind, persimmon, green tea, and black tea can be preferably used.

To be specific, epigallocatechin gallate (EGCG) can be prepared by, for example, purifying an extract from green tea leaves (cf. Japanese Patent Application Publication No. JP 2001-97968, etc.). Highly purified EGCG extracts like Teavigo® (DSM Nutrition Japan K.K.) may be used.

Also, OPCs can be prepared by, for example, purifying an extract from grape seeds (cf. Japanese Patent Domestic Publication No. JP 2009-502825, etc.). Further, Japanese Patent Application Publication No. JP H10-236943 discloses that an extract from *Jatobá* (West Indian locust) contains a condensed tannin with a degree of polymerization of 12.

An OPC-containing grape seed extract may be used, as it is, in the inventive composition and in the inventive agents for promoting the absorption of hydroxytyrosol in the body or for prolonging its residence time in the body. In such a case, commercially available grape seed extracts containing a high concentration of OPCs may be used, as used in working examples described later. Although commercially available grape seed extract products may vary slightly from lot to lot in terms of the content of a galloyl group in the OPCs contained in these products, the effects of this invention can be produced by using any of those products.

<Composition comprising hydroxytyrosol and a compound having a galloyl group>

According to the present invention, hydroxytyrosol or a derivative thereof, and a compound having a galloyl group are used in combination to thereby improve the absorption in the body of the hydroxytyrosol or the derivative thereof and prolong its residence time in the body, so that the physiological activities of the hydroxytyrosol or the derivative thereof can be exerted continuously and effectively, and that medical treatments and health promotion can be achieved by the physiological effects produced by the individual components.

In the inventive composition comprising hydroxytyrosol or a derivative thereof, and a compound having a galloyl group, the relative amounts and ratio of the hydroxytyrosol or the derivative thereof and the compound having a galloyl group are not particularly limited as long as the absorption in the body of the hydroxytyrosol or the derivative thereof can be promoted and its physiological activities can be exerted continuously, and said amounts and ratio can he selected as appropriate defending on various conditions including the form of the composition and pathological condition(s) to be treated, and other factors including the relationship of these components with other components to be selected.

For example, in the case where the composition of the present invention is a pharmaceutical composition, the pharmaceutical composition contains hydroxytyrosol, or a derivative thereof in a relative amount of preferably from 0.01 to 10% by weight, more preferably from 0.1 to 5% by weight, still more preferably from 0.1 to 1% by weight, and also contains a compound having a galloyl group in a relative amount of preferably from 0.1 to 30% by weight, more preferably from 0.5 to 20% by weight, still more preferably from 1 to 10% by weight. In the case where the compound having a galloyl group is a galloyl ester, the pharmaceutical composition contains the hydroxytyrosol, or the derivative thereof in a relative amount of preferably from 0.01 to 10% by weight, more preferably from 0.1 to 5% by weight, still more preferably from 0.1 to 1% by weight, and also contains the galloyl ester in a relative amount of from 0.01 to 10% by weight, more

preferably from 0.01 to 5% by weight, still more preferably from 0.01 to 1% by weight, in terms of the weight of a gallic acid produced by acid hydrolysis of the galloyl ester. In such a case, the relative ratio of the hydroxytyrosol or the derivative thereof and the compound having a galloyl group, which are contained in the pharmaceutical composition, is not limited, but if the promoting effect on the absorption in the body of the hydroxytyrosol, or the derivative thereof and the prolonging effect on its residence time in the body are expected to be produced, the relative ratio by weight of these components is preferably at least 1:0.1, more preferably in the range of from 1:0.1 to 1:100, still more preferably in the range of from 1:0.1 to 1:30, yet more preferably in the range of from 1:1 to 1:25. For example, the pharmaceutical composition of this invention can be a composition comprising the hydroxytyrosol or the derivative thereof, and the compound having a galloyl group at a weight ratio of from 1:0.1 to 1:100. Also, the inventive pharmaceutical composition can be a composition comprising the hydroxytyrosol or the derivative thereof, and the compound having a galloyl group at a weight ratio of from 1:0.1 to 1:30. Further, the inventive pharmaceutical composition can be a composition comprising the hydroxytyrosol or the derivative thereof, and an oligomeric proanthocyanidin at a weight ratio of from 1:1 to 1:100.

Further, the hydroxytyrosol or the derivative thereof, and the compound having a galloyl group are contained in the pharmaceutical composition at a molar ratio of preferably at least 1:0.1, more preferably from 1:0.1 to 1:10, still more preferably from 1:0.1 to 1:5, as calculated in terms of the ratio of the number of moles of hydroxytyrosol to that of a gallic acid produced by acid hydrolysis of the compound having a galloyl group. In the case where a catechin such as epigallocatechin gallate is used as the compound having a galloyl group, the hydroxytyrosol or the derivative thereof, and the catechin are contained in the pharmaceutical composition at a molar ratio of preferably at least 1:0.1, more preferably from 1:0.1 to 1:10, still more preferably from 1:0.1 to 1:5, as calculated in terms of the ratio of the number of moles of hydroxytyrosol to that of a gallic acid produced by acid hydrolysis of the catechin.

Furthermore, the pharmaceutical composition of the present invention can comprise an OPC in a relative amount of preferably from 0.1 to 30% by weight, more preferably from 0.5 to 20% by weight, still more preferably from 1 to 10% by weight.

The European Food Safety Authority (EFSA) reports that in order to obtain the physiological effects of hydroxytyrosol, or a derivative thereof, it is beneficial to consume the hydroxytyrosol or the derivative thereof generally in an amount of 5 mg per day for an adult individual. Therefore, it is preferred that hydroxytyrosol or a derivative thereof be incorporated in the inventive composition in such an amount that an adult individual can consume about 5 to 10 mg of hydroxytyrosol or a derivative thereof per day.

Thus, the composition of the present invention can promote the absorption in the body of hydroxytyrosol or a derivative thereof and prolong its residence time in the body. These effects can be determined by measuring the blood concentration of hydroxytyrosol or a derivative thereof, as described in Examples 1, 2, 3 and 4.

As described in detail below in the Examples section, the present inventors demonstrated that administration of 50 mg/kg of hydroxytyrosol (expressed as an amount (mg) of hydroxytyrosol per kg body weight of animal model) in combination with 150 mg/kg of epigallocatechin gallate significantly enhances the absorption of the hydroxytyrosol in the body, as compared to the case of administration of hydroxytyrosol alone. The inventors also confirmed that when consumed concurrently with epigallocatechin gallate, hydroxytyrosol has a unique absorption profile in terms of not only absorbed amount but also absorption rate. More specifically, as compared to the case of consumption of hydroxytyrosol alone, when hydroxytyrosol was consumed concurrently with EGCG, the blood hydroxytyrosol concentration showed a delay in reaching its peak and besides was kept high even thereafter. However, no similar results were obtained in the case where hydroxytyrosol was consumed concurrently with epigallocatechin which has no galloyl group; and this fact suggested that the galloyl group present in the structure of epigallocatechin gallate might be involved in this result. From this, it is conceivable that compounds having a galloyl group play a role in improving the absorption of hydroxytyrosol in the body. In particular, epicatechin gallate (ECG), gallocatechin gallate (GCG), and catechin gallate (CG), which are similar catechins having a similar structure to EGCG, can also be used similarly in this invention. By using the inventive agent for promoting absorption in the body, the blood hydroxytyrosol concentration can be maintained for a specified period of time, so that the effects of hydroxytyrosol can be exerted continuously for a long time. Further, the inventors confirmed that when rats were administered 30 mg/kg of hydroxytyrosol in combination with 500 mg/kg of a commercially available grape seed extract, the absorption of hydroxytyrosol, in the body at 0 to 6 hours was enhanced significantly, as compared to the case of administration of hydroxytyrosol alone. It was also found that when hydroxytyrosol was consumed concurrently with the grape seed extract, hydroxytyrosol was detected in blood and its blood concentration was maintained at a certain level even at 2 or 3 hours after administration, at which time little or no hydroxytyrosol was detected in blood when consumed alone.

Furthermore, the present inventors confirmed that when rats were administered 30 mg/kg of hydroxytyrosol in combination with 150 mg/kg or 250 mg/kg of the aforementioned grape seed extract, the absorption of hydroxytyrosol in the body at 0 to 6 hours was also enhanced, as compared to the case of administration of hydroxytyrosol alone.

Still furthermore, the present inventors confirmed that when rats were administered 30 mg/kg of hydroxytyrosol in combination with 150 mg/kg, 250 mg/kg, 500 mg/kg or 750 mg/kg of the aforementioned grape seed extract, the absorption of hydroxytyrosol in the body at 0 to 6 hours was also enhanced, as compared to the case of administration

of hydroxytyrosol alone.

<Agents for promoting the absorption of hydroxytyrosol in the body or for prolonging its residence time in the body>

As described above, the absorption of hydroxytyrosol, in the body can be enhanced by using hydroxytyrosol in combination with a compound having a galloyl group, such as epigallocatechin gallate or an OPC-rich grape seed extract. Also, the residence time of hydroxytyrosol in the body can be prolonged by using hydroxytyrosol in combination with a compound having a galloyl group, such as epigallocatechin gallate or an OPC-rich grape seed extract. Therefore, the agents according to the present invention can be used as agents for promoting absorption in the body of hydroxytyrosol or a derivative thereof and/or for prolonging its residence time in the body. The promoting effect on the absorption in the body of hydroxytyrosol or a derivative thereof according to this invention refers to the effect in which the amount of hydroxytyrosol, or a derivative thereof absorbed in the body can be increased as compared to the case of administration of hydroxytyrosol or a derivative thereof alone. This effect can be specifically exemplified by the increasing effect on the AUC (area under the blood concentration-time curve) of hydroxytyrosol or a derivative thereof. Also, the prolonging effect on the residence time in the body of hydroxytyrosol or a derivative thereof according to this invention refers to the effect in which the time period during which hydroxytyrosol or a derivative thereof can stay in the body without being degraded or metabolized can be prolonged as compared to the case of administration of hydroxytyrosol or a derivative thereof alone. This effect can be specifically exemplified by the sustaining effect on the blood concentration of hydroxytyrosol or a derivative thereof. The increasing effect on the AUC of hydroxytyrosol or a derivative thereof, and the sustaining effect on the blood concentration of hydroxytyrosol or a derivative thereof can be evaluated by measuring the blood concentration of hydroxytyrosol or a derivative thereof with the use of a means known to those skilled in the art.

The inventive agents for promoting absorption in the body of hydroxytyrosol or a derivative thereof or for prolonging its residence time in the body comprise a compound having a galloyl group in a relative amount of preferably from 0.1 to 30% by weight, more preferably from 0.5 to 20% by weight, still more preferably from 1 to 10% by weight.

In the case where the compound having a galloyl group is a galloyl ester which produces a gallic acid by acid hydrolysis, the inventive agents for promoting absorption in the body of hydroxytyrosol or a derivative thereof or for prolonging its residence time in the body comprise the galloyl ester in a relative amount of preferably from 0.01 to 25% by weight, more preferably from 0.01 to 10% by weight, still more preferably from 0.01 to 1% by weight, in terms of the weight of a gallic acid produced by acid hydrolysis of the galloyl ester. Also, in the case where the compound having a galloyl group is the galloyl ester which produces a gallic acid by acid hydrolysis, the inventive agents for promoting absorption in the body of hydroxytyrosol or a derivative thereof or for prolonging its residence time in the body comprise, per gram of the agent, the galloyl ester in a relative amount of preferably from 0.01 to 1 mmol, more preferably from 0.05 to 0.5 mmol, still more preferably from 0.05 to 0.3 mmol, in terms of the gallic acid produced by acid hydrolysis of the galloyl ester.

Further, in the case where the compound having a galloyl group is an OPC, the inventive agents for promoting absorption in the body of hydroxytyrosol or a derivative thereof or for prolonging its residence time in the body comprise the OPCs in a relative amount of preferably from 0.1 to 30% by weight, more preferably from 0.5 to 20% by weight, still more preferably from 1 to 10% by weight.

In the case where the inventive agents for promoting absorption in the body of hydroxytyrosol or a derivative thereof or for prolonging its residence time in the body are made into, for example, a pharmaceutical product, the agents can be formulated with a pharmaceutically acceptable base or carrier and provided as a pharmaceutical composition. In addition to such a base or carrier, the pharmaceutical composition may have added thereto in any desired amounts any additives, including binders, disintegrants, buffers, preservatives, humectants, anti-microbials, anti-septics, flavorants, surfactants, stabilizers and solubilizers, as long as such additives are pharmaceutically acceptable. The pharmaceutical composition may be administered in an oral dosage form or in the form of parenteral injection or the like, and any dosage form known as a suitable form for the mode of administration selected can be used as appropriate. Examples of the dosage form suitable for oral administration include, but are not limited to, tablet, capsule, powder, granule, solution, suspension and syrup.

## EXAMPLES

The present invention will be described in more detail with reference to the working examples given below, but these examples do not limit the scope of this invention. Those skilled in the art could use this invention with various alterations or modifications being made thereto, and such alterations and modifications are also included in the scope of this invention.

Example 1:

The present inventors analyzed the influence of the particular compound having a galloyl group, *i.e.,* EGCG, on the absorption of hydroxytyrosol, in the body. At the same time, the inventors also analyzed the influence of EGC, a compound having no galloyl group, on the absorption of hydroxytyrosol in the body. By comparing the results for EGCG with those for the control, we investigated whether the galloyl group is involved in the absorption of hydroxytyrosol in the body.

SD (IGS) male rats (5 weeks old) were purchased from Oriental Bioservice, Inc. and habituated to the test environment for one week, and then those rats which were well grown were put to use in the selected test. Those rats fasted overnight were divided into three groups each consisting of 4 or 5 animals. Group 1 (control) was given 5 mL/kg of distilled water and 50 mg/5 mL/kg of a commercial hydroxytyrosol solution in distilled water; Group 2 was given 100 mg/5 mL/kg of an EGC solution in distilled water and 50 mg/5 mL/kg of a hydroxytyrosol solution in distilled water; and Group 3 was given 150 mg/5 mL/kg of an EGCG solution in distilled water and 50 mg/5 mL/kg of a hydroxytyrosol solution in distilled water -- all of the groups were orally administered using a sonde. Before and at 0.25, 0.5, 1, 3 and 6 hours after the start of treatment, blood was sampled from the tail vein using a heparin-coated blood collection tube and centrifuged (at 8000 rpm for 10 min) to obtain plasma samples. The obtained plasma samples were subjected to deconjugation and deproteinization treatments, and the resulting supernatants were concentrated under reduced pressure, redissolved in 20% acetonitrile, and passed through a filter. The resulting solutions were analyzed by HPLC to determine their peak areas. Thus, quantification of hydroxytyrosol was performed. The EGCG used in this example was a commercially available EGCG product (EGCG purity: 94 %). The HPLC conditions are shown below.

Column: X-bridge C18 (3.5 μm, 2.1×150 mm, produced by Waters)

Mobile phase: A: 0.1% acetic acid solution in water, B: 0.1% acetic acid/90% acetonitrile solution in water

Flow rate: 0.25 mL/min

Gradient program:

5 → 30% B (0-10 min)
30 → 85% (10-11 min)
85% (11-15 min)
85 to 5% (15-15.1 min)
5% (15.1-20 min)

FIG. 1 shows the amount of hydroxytyrosol absorbed in the body (AUC). The AUC for the control treated with hydroxytyrosol alone was 12.05 μ.g/mL*hr, whereas when hydroxytyrosol was administered concurrently with 150 mg/5 mL/kg of the EGCG solution in distilled water, the AUC of hydroxytyrosol, was increased to 16.63 μg/mL*hr. In this case, the molar ratio of the hydroxytyrosol administered, relative to a gallic acid present in the EGCG administered, was 1:1.

However, the AUC in the case of administration of hydroxytyrosol concurrently with 100 mg/5 mL/kg of the EGC solution in distilled water was 12.13 μg/mL*hr, which is almost the same value as the AUC for the control. These results suggested that EGC, which has no galloyl group, has no influence on the absorption of hydroxytyrosol.

FIG. 2 shows the transition of blood hydroxytyrosol concentration. When consumed concurrently with EGCG. hydroxytyrosol showed a unique absorption profile. More specifically, when Hydroxytyrosol was consumed alone, the blood hydroxytyrosol concentration reached its peak 18 minutes after the treatment and then decreased soon; on the other hand, when hydroxytyrosol was consumed concurrently with EGCG, the blood hydroxytyrosol concentration reached its peak 30 minutes after the treatment and was kept high even thereafter.

In contrast, the absorption profile of hydroxytyrosol when consumed concurrently with EGC was almost the same as that for the control.

These results revealed that when hydroxytyrosol is consumed concurrently with EGCG, the absorption of hydroxytyrosol in the body is improved and its residence time in the body is prolonged. Also, judging from the fact that no similar effects were observed in the case of concurrent administration with EGC, it is considered that a galloyl group present in the structure of EGCG is involved in the improvement of the absorption of hydroxytyrosol in the body. From this, it is conceivable that compounds having a galloyl group play a role in improving the absorption of hydroxytyrosol in the body. ECG, GCG and CG, which are similar catechins having a similar structure to EGCG, can also be used similarly.

Example 2:

The present inventors investigated the influence of an OPC on the absorption of hydroxytyrosol in the body.

SD (IGS) male rats (9 weeks old) were purchased from Oriental Bioservice, Inc. and habituated to the test environment for one week, and then those rats which were well grown were put to use in the selected test. Those rats fasted overnight were divided into two groups each consisting of 5 or 6 animals. Group 1 (control) was given 30 mg/5 mL/kg of a hydroxytyrosol solution in distilled water; and Group 2 was given 30 mg/kg of hydroxytyrosol plus 5 mL/kg of a solution of 500 mg/kg grape seed extract in distilled water -- both of the groups were orally administered using a sonde. Before and at 0.25, 0.5, 1, 2, 3 and 6 hours after the start of treatment, blood was sampled from the tail vein using a heparin-coated blood collection tube and centrifuged (at 8000 rpm for 10 min) to obtain plasma samples. The obtained plasma samples were subjected to deconjugation and deproteinization treatments, and the resulting supernatants were concentrated under reduced pressure, redissolved in 20% acetonitrile, and passed through a filter. The resulting solutions were analyzed by LC/MS/MS, and quantification of hydroxytyrosol was performed using the internal standard method.

HPLC conditions

Internal standard (I.S.): 3-(4-hydroxyphenyl)-1-propanol
Column: Capcell Pak, AQ, C18 (3.0 μm, 2.0×100 mm, produced by Shiseido Japan Co., Ltd.)
Mobile phase: A: 0.1% acetic acid solution in water, B: 0.1% acetic acid/90% acetonitrile solution in water
Flow rate: 0.25 mL/min
Gradient program:

5 → 30% B (0-10 min)
30 → 85% (10-11 min)
85% (11-15 min)
85 → 5% (15-15.1 min)
5% (15.1-20 min)
MS conditions
Ionization: ESI
IS: -4500 V
TEM: 600°C
GS1: 60 psi
GS2: 80 psi
CUR: 50 psi
CAD: 7

Detection: Negative ion MRM

[Table 1]

| Object to be analyzed | Detected ion | DP (V) | EP (V) | CE (V) | CXP (V) |
|---|---|---|---|---|---|
| Hydroxytyrosol | *m/z* 153 → *m/z* 123 | -60 | -10 | -20 | -1 |
| I.S. | *m/z* 151 → *m/z* 106 | -60 | -10 | -22 | -5 |

Analysis time: 20 min

FIG. 3 shows the absorption of hydroxytyrosol, in the body (AUC), and FIG. 4 shows the transition of blood hydroxytyrosol concentration. The AUC at 0 to 6 hours for the control treated with hydroxytyrosol alone was 6.21 μg/mL*hr, whereas when hydroxytyrosol was administered concurrently with the grape seed extract solution in distilled water, the AUC of hydroxytyrosol at 0 to 6 hours was increased to 12.06 μg/mL*hr.

These results revealed that when hydroxytyrosol is consumed concurrently with an OPC, the absorption of hydroxytyrosol in the body is improved.

Example 3:

The present inventors investigated whether the same promoting effect on the absorption of hydroxytyrosol in the body as observed in Example 2 can be observed even if the dose of the grape seed extract is reduced.

SD (IGS) male rats (9.5 weeks old) were purchased from Oriental Bioservice, Inc. and habituated to the test environment for two weeks, and then those rats which were well grown were put to use in the selected test. Those rats fasted overnight were divided into three groups each consisting of 4 animals. Group 1 (control) was given 30 mg/5 mL/kg of a hydroxytyrosol solution in distilled water; Group 2 was given 30 mg/kg of hydroxytyrosol plus 5 mL/kg of a solution of 150 mg/kg grape seed extract in distilled water; and Group 3 was given 30 mg/kg of hydroxytyrosol plus 5 mL/kg of a solution of 250 mg/kg grape seed extract in distilled water -- all of the groups were orally administered using a sonde. Before and at 0.25, 0.5, 1, 2, 3 and 6 hours after the start of treatment, blood was sampled from the tail vein using a heparin-coated blood collection tube and centrifuged (at 8000 rpm for 10 min) to obtain plasma samples. The grape seed extract used in this example was a commercially available product produced by the same manufacturer as the product used in Example 2. The obtained plasma samples were subjected to deconjugation and deproteinization treatments, and the resulting supernatants were concentrated under reduced pressure, redissolved in 20% acetonitrile, and passed through a filter. The resulting solutions were analyzed by LC/MS/MS, and quantification of hydroxytyrosol was performed using the internal standard method. The LC and MS were performed under the same conditions as in Example 2.

FIG. 5 shows the absorption of hydroxytyrosol in the body (AUC). The AUC at 0 to 6 hours for the control treated with hydroxytyrosol alone was 8.64 μg/mL*hr, whereas when hydroxytyrosol was consumed concurrently with the solution of 150 mg/kg or 250 mg/kg grape seed extract in distilled water, the AUCs of hydroxytyrosol, at 0 to 6 hours were increased to 13.41 and 17.52 μg/mL*hr, respectively. These results revealed that the absorption of hydroxytyrosol in the body is improved even when hydroxytyrosol is administered concurrently with 150 mg/kg or 250 mg/kg of a grape seed extract.

Example 4:

The present inventors investigated whether the same promoting effect on the absorption of hydroxytyrosol in the body can be observed even if the dose of the grape seed extract is further increased in the same test system as used for the investigations in Examples 2 and 3. The grape seed extract used for this investigation contained OPCs composed of a dimer and higher polymers and had an OPC purity of about 76.6% as calculated in terms of procyanidin B1. The method for calculating this purity will be presented later in Example 5. Also, the amount of a galloyl group contained in the grape seed extract was measured as the amount of a galloyl ester and determined to be about 2.2% by weight. The method for calculating this amount will be presented later in Example 6.

SD (IGS) male rats (8 weeks old) were purchased from Oriental Bioservice, Inc. and habituated to the test environment for one week, and then those rats which were well grown were put to use in the selected test. Those rats fasted overnight were divided into five groups each consisting of 4 animals (however, Group 5 consists of 2 animals). Group 1 (control) was given 30 mg/5 mL/kg of a hydroxytyrosol, solution in distilled water; Group 2 was given 30 mg/kg of hydroxytyrosol plus 5 mL/kg of a solution of 150 mg/kg grape seed extract in distilled water; Group 3 was given 30 mg/kg of hydroxytyrosol plus 5 mL/kg of a solution of 250 mg/kg grape seed extract in distilled water; Group 4 was given 30 mg/kg of hydroxytyrosol plus 5 mL/kg of a solution of 500 mg/kg grape seed extract in distilled water; and Group 5 was given 30 mg/kg of hydroxytyrosol plus 5 mL/kg of a solution of 750 mg/kg grape seed extract in distilled water -- all of the groups were orally administered using a sonde. Before and at 0.25, 0.5, 1, 2, 3, 6 and 8 hours after the start of treatment, blood was sampled from the tail vein using a heparin-coated blood collection tube and centrifuged (at 8000 rpm for 10 min) to obtain plasma samples.

The obtained plasma samples were subjected to deconjugation and deproteinization treatments, and the resulting supernatants were concentrated under reduced pressure, redissolved in 20% acetonitrile, and passed through a filter. The resulting solutions were analyzed by LC/MS/MS, and quantification of hydroxytyrosol, was performed using the internal standard method. The LC and MS were performed under the same conditions as in Example 2.

FIG. 6 shows the absorption of hydroxytyrosol in the body (AUC). The AUC at 0 to 6 hours for the control treated with hydroxytyrosol alone was 7.36 μg/mL*hr, whereas when hydroxytyrosol was consumed concurrently with the solution of 150 mg/kg, 250 mg/kg, 500 mg/kg or 750 mg/kg grape seed extract in distilled water, the AUCs of hydroxytyrosol at 0 to 6 hours were increased to 9.89, 9.68, 11.22 and 12.86 μg/mL*hr, respectively. These results revealed that the absorption of hydroxytyrosol in the body is improved when hydroxytyrosol is administered concurrently with 150 mg/kg, 250 mg/kg, 500 mg/kg or 750 mg/kg of a grape seed extract. The amounts of a galloyl ester in the grape seed extracts (150 mg/kg, 250 mg/kg, 500 mg/kg and 750 mg/kg) used in this example were 3.3 mg/kg, 5.5 mg/kg, 11 mg/kg and 16.5 mg/kg. respectively, and the molar ratios of HT and the galloyl ester were 1:0.1, 1:0.17, 1:0.34 and 1:0.51, respectively. Also, the amounts of OPCs in 150 mg/kg, 250 mg/kg, 500 mg/kg and 750 mg/kg of the grape seed extracts used in this

example were 114.9 mg/kg, 191.5 mg/kg, 383 mg/kg and 574.5 mg/kg, respectively, and the HT/OPCs weight ratios were 1:3.83, 1:6.38, 1:12.77 and 1:19.15. respectively.

FIG. 7 shows the transition of blood hydroxytyrosol (HT) concentration. It was demonstrated that when hydroxytyrosol, is consumed concurrently with a grape seed extract, the absorption of hydroxytyrosol in the body is improved and its residence time in the body is prolonged.

Example 5: Determination of the purity of oligomeric proanthocyanidins in a grape seed extract

The purity of oligomeric proanthocyanidins derived from a grape seed extract was determined pursuant to the method described in Japanese Patent No. JP 4659407. To be specific, the purity determination was performed according to the procedure described below.

First, 1.0 mL of 0.6 N HCl/butanol was added to 1.0 mg of the grape seed extract prepared in the above examples, and the mixture was reacted at 90°C for two hours to decompose oligomeric proanthocyanidins into cyanidin. The resulting reaction solution was analyzed under the HPLC conditions described below to quantify the content of cyanidin in the reaction solution. Then, the purity of oligomeric proanthocyanidins was calculated using the calculation equation presented below. The internal standard used was proanthocyanidin B-1 (PB-1) (NIU-N210, produced by Funakoshi Co., Ltd.).

HPLC conditions

Detection wavelength: 520 nm
Column: YMC-Pack ODS A-312 (product name, φ6.0×150.0 mm, produced by YMC Co., Ltd.)
Solvent: Water/methanol/acetic acid = 67.5:17.5:15.0 (volume ratio)
Column temperature: 40°C
Flow rate: 1.0 mL/min

Equation for calculating OPCs purity:

[Formula 1]

$$\text{OPC purity (\%)} = \frac{\text{Cyanidin concentration in sample}}{\text{Cyanidin concentration in PB-1}} \times 100$$

The purity of oligomeric proanthocyanidins in the grape seed extract used in Example 4 was calculated using the aforementioned equation and determined to be about 76.6%.

Example 6: Determination of the galloyl ester content in a grape seed extract

A screw-top test tube was charged with 0.5 mL of a solution of a grape seed extract adjusted with distilled water to 4 mg/mL, and 0.5 mL of a 10% sulfuric acid solution in water (v/v), and was stirred, closed with a screw cap, and treated at 90°C for 14 hours. After the resulting reaction solution was cooled, 0.5 mL of dimethylsulfoxide was added to 0.5 mL of the reaction solution. The mixture was stirred and then analyzed under the high performance liquid chromatography conditions described below to determine a galloyl ester content.

HPLC conditions

Detection wavelength: 280 nm
Column: Develosil C30 (φ4.6× 150 mm, produced by Nomura Chemical Co., Ltd.)
Solvent A: 0.05% trifluoroacetic acid-containing solution in water
Solvent B: 0.05% trifluoroacetic acid-containing solution of 90% acetonitrile in water
Gradient: Solvent B: 6% (0 min) → 6% (5 min) → 17% (11 min) → 19% (21 min) → 88% (22 min) → 88% (35 min)

Column temperature: 40°C
Flow rate: 1.0 mL/min

The concentration of a galloyl ester was calculated according to the equation mentioned below.

Equation for calculating galloyl ester content

$$\text{Gallic acid content after reaction} - \text{Gallic acid content before reaction} = \text{Galloyl ester content}$$

The content of a gallic acid in the grape seed extract used in Example 4 was 28 μg/mg after reaction and 5.9 μg/mg before reaction. Based on these values, the galloyl ester content in the grape seed extract was determined to be 22.1 μg/mg, and the relative proportion of the galloyl ester in the grape seed extract to be about 2.2%.

INDUSTRIAL APPLICABILITY

The present invention is useful for increasing the rate of utilization in the body of hydroxytyrosol which has beneficial activities for the living body. This invention is also useful for producing and developing foods and beverages or pharmaceutical products which are beneficial for inflammatory and other diseases and for anti-aging.

## Claims

**1.** A composition comprising hydroxytyrosol or a derivative thereof, and a compound having a galloyl group.

**2.** The composition as set forth in claim 1, wherein the compound having a galloyl group is a monomer of a flavane compound having one or more galloyl groups in a flavane backbone.

**3.** The composition as set forth in claim 2, wherein the monomer is a flavane compound having a galloyl group attached to position 3 of the flavane backbone.

**4.** The composition as set forth in claim 2, wherein the compound having a galloyl group is a catechin compound having a galloyl group.

**5.** The composition as set forth in claim 4, wherein the catechin compound having a galloyl group is a catechin compound comprising at least one member selected from the group consisting of catechin gallate, epicatechin gallate, gallocatechin gallate, epigallocatechin gallate and methylated derivatives thereof.

**6.** The composition as set forth in claim 1, wherein the compound having a galloyl group is a polymer of a flavane compound having a flavane backbone, wherein at least one monomeric unit is a flavane compound having a galloyl group attached to position 3 of the flavane backbone.

**7.** The composition as set forth in claim 6, wherein the polymer is an oligomeric proanthocyanidin (hereinafter also referred to as simply "proanthocyanidin" or as "OPC").

**8.** The composition as set forth in any one of claims 1 to 7, wherein the compound having a galloyl group is derived from grape, pine, aronia, peanut, cocoa, apple, red bean, tamarind, persimmon, green tea, or black tea.

**9.** The composition as set forth in claim 8, wherein the compound having a galloyl group is derived from a grape seed extract.

**10.** A composition comprising hydroxytyrosol or a derivative thereof, and a grape seed extract.

**11.** The composition as set forth in any one of claims 1 to 9, wherein the hydroxytyrosol or the derivative thereof, and the compound having a galloyl group are present at a weight ratio of from 1:0.1 to 1:100.

**12.** The composition as set forth in claim 11, wherein the hydroxytyrosol or the derivative thereof, and the compound having a galloyl group are present at a weight ratio of from 1:0.1 to 1:30.

**13.** The composition as set forth in claim 11 or 12, wherein the compound having a galloyl group is a monomer of a flavane compound having one or more galloyl groups in a flavane backbone.

**14.** The composition as set forth in claim 11, wherein the hydroxytyrosol, or the derivative thereof, and the oligomeric proanthocyanidins are present at a weight ratio of from 1:1 to 1:100.

**15.** The composition as set forth in any one of claims 1 to 9, wherein the compound having a galloyl group is a galloyl ester which produces a gallic acid by acid hydrolysis, and wherein the hydroxytyrosol or the derivative thereof, and the gallic acid produced by acid hydrolysis of the galloyl ester are present at a molar ratio of from 1:0.1 to 1:10.

**16.** The composition as set forth in any one of claims 1 to 10, wherein the hydroxytyrosol or the derivative thereof is present in a relative amount of from 0.01 to 10% by weight, and the compound having a galloyl group is present in a relative amount of from 0.1 to 30% by weight.

**17.** The composition as set forth in any one of claims 1 to 16, wherein the composition is a food or beverage.

**18.** The composition as set forth in any one of claims 1 to 16, wherein the composition is a pharmaceutical composition.

**19.** An agent for promoting absorption in the body, and/or for prolonging residence time in the body, of hydroxytyrosol or a derivative thereof, the agent comprising, as an active component, a compound having a galloyl group.

**20.** The agent as set forth in claim 19, wherein the compound having a galloyl group is a monomer of a flavane compound having one or more galloyl groups in a flavane backbone.

**21.** The agent as set forth in claim 20, wherein the monomer is a flavane compound having a galloyl group attached to position 3 of the flavane backbone.

**22.** The agent as set forth in claim 20, wherein the compound having a galloyl group is a catechin compound having a galloyl group.

**23.** The agent as set forth in claim 22, wherein the catechin compound having a galloyl group is a catechin compound comprising at least one member selected from the group consisting of catechin gallate, epicatechin gallate, gallocatechin gallate, epigallocatechin gallate and methylated derivatives thereof.

**24.** The agent as set forth in claim 19, wherein the compound having a galloyl group is a polymer of a flavane compound having a flavane backbone, wherein at least one monomeric unit is a flavane compound having a galloyl group attached to position 3 of the flavane backbone.

**25.** The agent as set forth in claim 24, wherein the polymer is an oligomeric proanthocyanidin.

**26.** The agent as set forth in any one of claims 19 to 25, wherein the compound having a galloyl group is derived from grape, pine, aronia, peanut, cocoa, apple, red bean, tamarind, persimmon, green tea, or black tea.

**27.** The agent as set forth in claim 26, wherein the compound having a galloyl group is derived from a grape seed extract.

**28.** An agent for promoting absorption in the body, and/or for prolonging residence time in the body, of hydroxytyrosol or a derivative thereof, the agent comprising a grape seed extract.

**29.** The agent as set forth in any one of claims 19 to 28, wherein the compound having a galloyl group is present in a relative amount of 0.1 to 30% by weight.

**30.** The agent as set forth in any one of claims 19 to 29, wherein the compound having a galloyl group is a galloyl ester which produces a gallic acid by acid hydrolysis, wherein the galloyl ester is present in a relative amount of 0.01 to 25% by weight in terms of the weight of the gallic acid produced by acid hydrolysis of the galloyl ester.

**31.** The agent as set forth in any one of claims 19 to 28, wherein the compound having a galloyl group is a galloyl ester which produces a gallic acid by acid hydrolysis, wherein the relative amount of the galloyl ester present per gram of the agent is in the range of from 0.001 to 1 mmol in terms of the gallic acid produced by acid hydrolysis of the galloyl ester.

**32.** The agent as set forth in any one of claims 25 to 29, wherein the oligomeric proanthocyanidin is present in a relative amount of 0.1 to 30% by weight.

FIG. 1

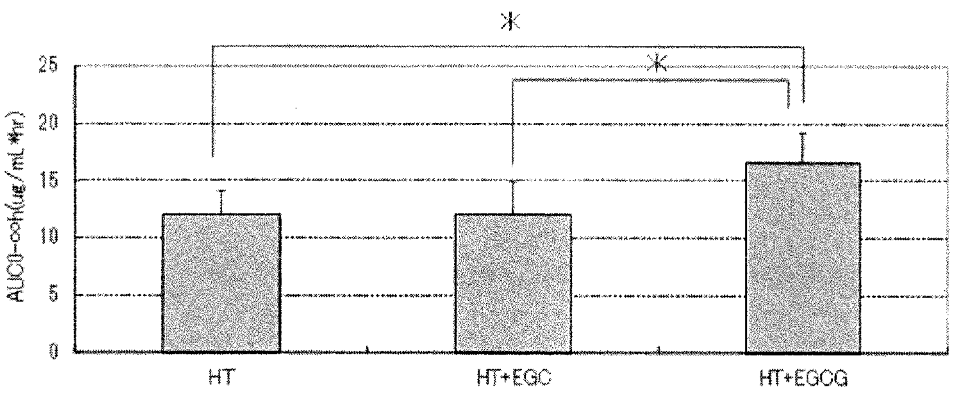


※ ; $p$< 0.05 (dunnett test)

* HT (Hydroxytyrosol)

Amount of hydroxytyrosol absorbed in the body (AUC)

FIG. 2

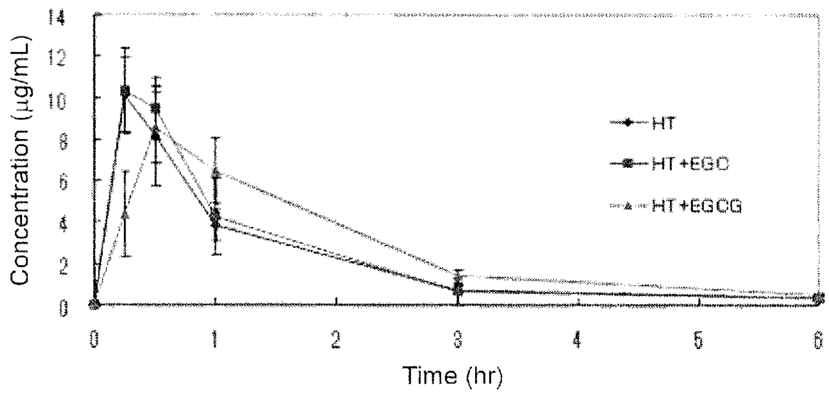


Transition of blood hydroxytyrosol concentration

FIG. 3

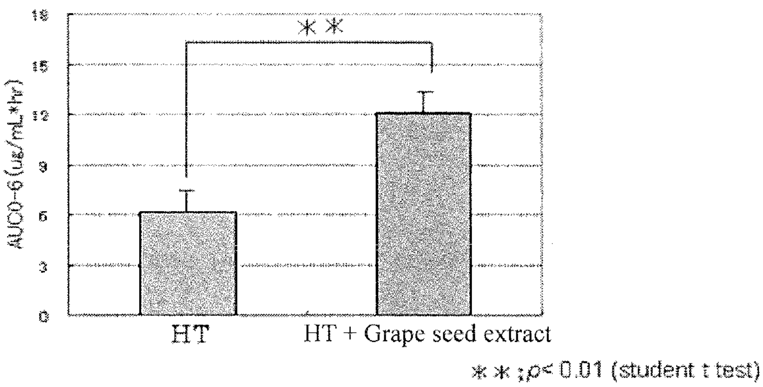


Amount of hydroxytyrosol absorbed in the body (AUC)

FIG. 4

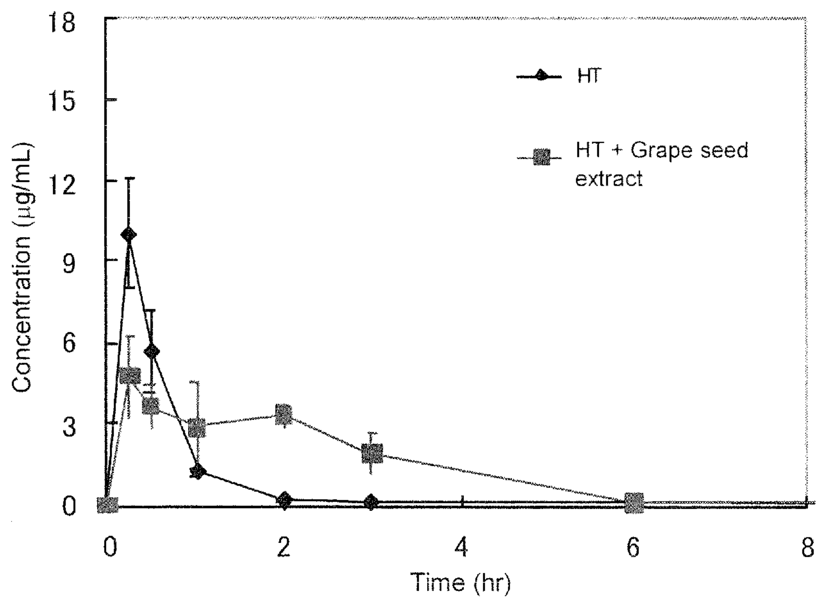


Transition of blood hydroxytyrosol concentration

*FIG. 5*

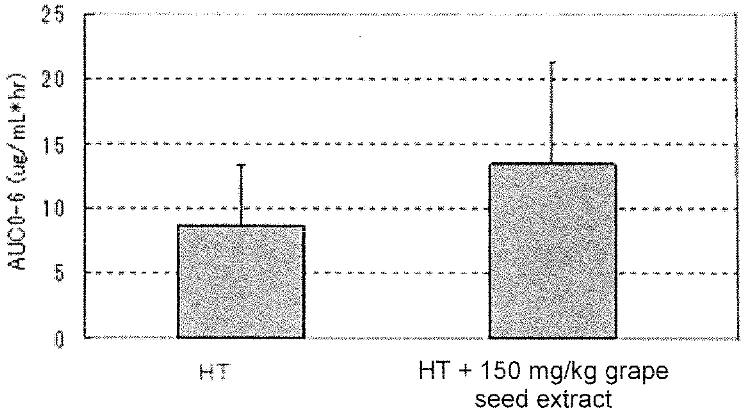


Amount of hydroxytyrosol absorbed in the body (AUC)

*FIG. 6*

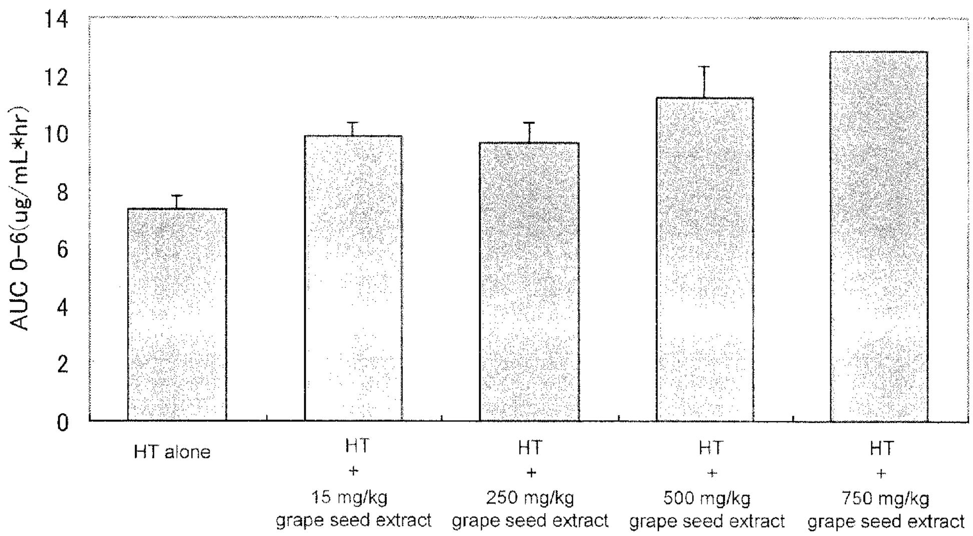


| | | | | | |
|---|---|---|---|---|---|
| HT/galloyl ester molar ratio | 1:0 | 1:0.1 | 1: 0.17 | 1:0.34 | 1:0.51 |
| HT/OPC weight ratio | 1:0 | 1:3.83 | 1: 6.38 | 1:12.77 | 1:19.15 |

* ;$p < 0.05$ (dunnett test)

Amount of hydroxytyrosol absorbed in the body (AUC)

FIG. 7

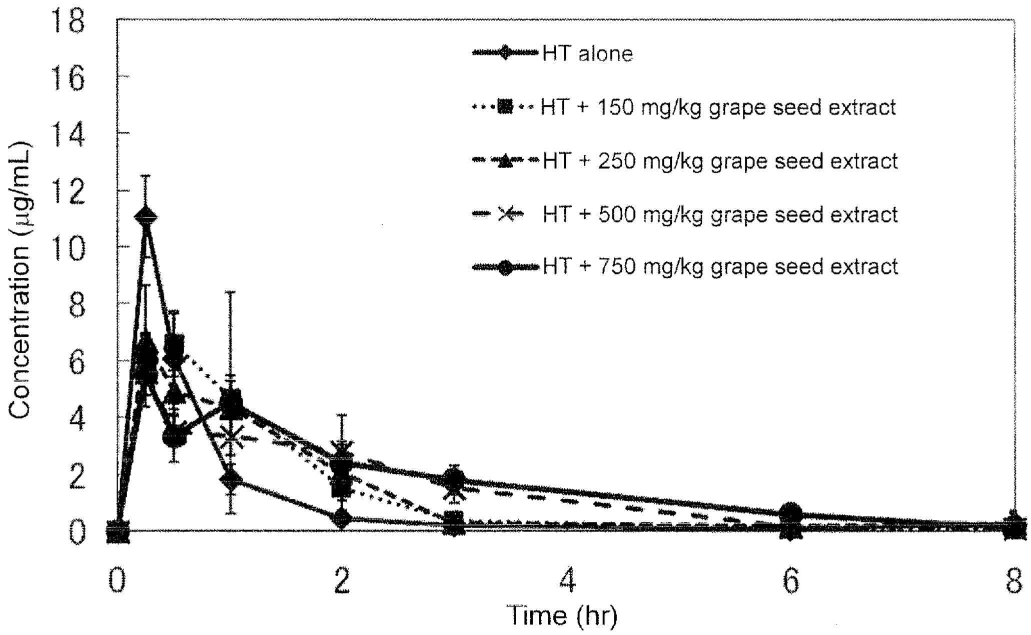


Transition of blood hydroxytyrosol concentration

## INTERNATIONAL SEARCH REPORT

International application No.
PCT/JP2014/070839

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
A61K31/05, A23L1/30, A61K31/353, A61K36/00, A61K36/18, A61K36/48, A61K36/73, A61P3/06, A61P3/10, A61P9/12, A61P29/00, A61P31/04, A61P39/06, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY/MEDLINE/EMBASE/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | JP 2010-500964 A (DSM IP Assets B.V.), 14 January 2010 (14.01.2010), claims; examples; table 2 & US 2010/0056463 A1 & EP 2040696 A2 & WO 2008/006581 A2 & KR 10-2009-0028836 A & CN 101516364 A | 1-5,8,11-13, 15-23,29-31 |
| X | JP 2009-511522 A (DSM IP Assets B.V.), 19 March 2009 (19.03.2009), claims; example 3 & US 2009/0163579 A1 & EP 1937232 A2 & WO 2007/042271 A2 & CN 101312718 A & KR 10-2008-0105023 A & CN 101978958 A | 1-5,8,11-13, 15-18 |

☒ Further documents are listed in the continuation of Box C.　　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 October, 2014 (28.10.14) | 11 November, 2014 (11.11.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No. PCT/JP2014/070839

C (Continuation). DOCUMENTS CONSIDERED TO BE RELEVANT

| Category* | Citation of document, with indication, where appropriate, of the relevant passages | Relevant to claim No. |
|---|---|---|
| X | JP 2010-502662 A (DSM IP Assets B.V.), 28 January 2010 (28.01.2010), claims; examples 2, 3 & US 2010/0080762 A1 & EP 1897530 A1 & WO 2008/028631 A2 | 1-5,8,11-13, 15-18 |
| X | JP 2008-094754 A (EN Otsuka Pharmaceutical Co., Ltd.), 24 April 2008 (24.04.2008), claims; example 1 (Family: none) | 1-18 |
| X | JP 2012-517824 A (Centre de Cooperation Internationale en Recherche Agronomique pour le Developpement (CIRAD)), 09 August 2012 (09.08.2012), claims; paragraphs [0063] to [0065]; example 5 & US 2012/0045406 A1 & EP 2398756 A2 & WO 2010/094860 A2 & FR 2942224 A1 & AR 75518 A & AU 2010215354 A | 1,6-12,14, 16-19,24-29, 32 |
| A | Pazos et al., Physiochemical properties of natural phenolics from grapes and olive oil byproducts and their antioxidant activity in frozen Horse mackerel fillets, Journal of Agricultural and Food Chemistry, (2006), 54, 366-373 | 1-32 |

Form PCT/ISA/210 (continuation of second sheet) (July 2009)

**INTERNATIONAL SEARCH REPORT**

International application No.
PCT/JP2014/070839

Continuation of A. CLASSIFICATION OF SUBJECT MATTER
(International Patent Classification (IPC))

*A61K31/05*(2006.01)i, *A23L1/30*(2006.01)i, *A61K31/353*(2006.01)i, *A61K36/00*(2006.01)i, *A61K36/18*(2006.01)i, *A61K36/48*(2006.01)i, *A61K36/73*(2006.01)i, *A61P3/06*(2006.01)i, *A61P3/10*(2006.01)i, *A61P9/12*(2006.01)i, *A61P29/00*(2006.01)i, *A61P31/04*(2006.01)i, *A61P39/06*(2006.01)i, *A61P43/00*(2006.01)i

(According to International Patent Classification (IPC) or to both national classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010052487 A
- JP 2010011751 A
- JP 2011079770 A
- JP 2001097968 A
- JP 2009502825 A
- JP H10236943 B
- JP 4659407 B